# FASCICULE DE BREVET EUROPEEN

(11) **EP 0 582 493 B1**
(45) Date de publication et mention de la délivrance du brevet: **30.07.1997**
(21) Numéro de dépôt: 93401598.3
(22) Date de dépôt: 22.06.1993
(51) Int. Cl.: A61F 2/02

(54) **Filtre à pattes triangulées**
Gefäss-Filter mit dreieckigen Schenkeln
Vascular filter with triangulated legs

(30) Priorité: 07.08.1992 FR 9209845
(43) Date de publication de la demande: 09.02.1994
(73) Titulaire: B. BRAUN CELSA, 86360 Chasseneuil du Poitou (FR)
(72) Inventeur: Cottenceau, Jean-Philippe, F-92160 Antony (FR); Chevillon, Gérard, F-92120 Montrouge (FR)
(74) Mandataire: Lerner, François

(56) Documents cités:
- EP-A- 0 117 940
- EP-A- 0 423 916
- EP-A- 0 430 848
- WO-A-89/08433
- DE-A- 3 417 738
- DE-A- 4 030 998
- FR-A- 2 525 896
- FR-A- 2 573 646

## Description

L'invention a pour objet un filtre destiné à être placé sur le trajet sanguin en particulier sur le trajet veineux pour la retenue de caillots sanguins comme défini dans le préambule de la revendication 1. Un tel filtre est connu du document DE-A-4 030 998.

Des filtres similaires sont par exemple décrits dans les brevets FR-A- 2 570 288 ou encore FR-A- 2 573 646.

De façon générale, ces filtres se présentent sous la forme d'un petit panier tronconique qui s'accroche à l'intérieur d'une veine en aval du trajet que l'on veut filtrer ; il s'agit en général de la veine cave arrivant au coeur. On peut ainsi arrêter, avant leur entrée dans le coeur, les éventuels caillots sanguins pouvant se former et risquant d'entraîner notamment des embolies.

Habituellement ces filtres sont de deux types principaux.

On distingue tout d'abord les filtres fabriqués à partir de fils métalliques assemblés entre eux par soudage ou sertissage, ils sont de petit diamètre et souples. Une fois repliés, ils sont peu encombrants et s'adaptent très bien à l'introduction par des voies difficiles et présentant des courbures anatomiques prononcées.

Par ailleurs, on distingue les filtres fabriqués à partir de plaques permettant généralement la réalisation de la partie filtrante en une seule pièce découpée et mise en forme ; ces filtres présentent une surface d'appui importante au contact de la veine réduisant ainsi les traumatismes et éliminant les risques de perforation.

Toutefois, ces deux types de filtres présentent divers inconvénients.

En effet, pour les filtres fabriqués avec des fils, la souplesse des fils génère deux défauts non négligeables :
- d'une part, elle empêche d'avoir des forces d'appui conséquentes sur la paroi de la veine, le filtre est mal maintenu et peut migrer.
- d'autre part, la dimension du fil est réduite ; la surface d'appui au contact de la veine est alors limitée ce qui peut entraîner des traumatismes et des perforations.

En outre, il existe un risque d'emmêlement des fils plus particulièrement au moment de la pose réduisant alors la capacité de filtration des filtres.

Les filtres fabriqués à partir de plaques présentent quant à eux :
- une rigidité importante dans leur position resserrée de mise en place qui complique leur pose dans des voies d'abord sinueuses.
- un encombrement non négligeable du fait des surfaces qui ne permet pas l'utilisation d'introducteurs très fins, augmentant ainsi le traumatisme au point de ponction.

Par ailleurs, les filtres formés d'une partie conique dotée d'éléments filtrants sont généralement difficiles à positionner correctement. D'une façon générale, pour introduire un tel filtre dans le vaisseau, on l'y pousse au moyen d'un tube dit cathéter qui traverse ledit vaisseau et dont le diamètre est inférieur à celui de ce dernier. Lorsque le filtre arrive à l'extrémité du tube d'introduction, il est alors lâché dans le vaisseau et l'expansion de ses pattes souvent munies de crochets assure son ancrage.

Un tel "lâché" est dans la pratique très délicat à contrôler, et il s'est avéré dans un certain nombre de cas que le filtre en panier occupait en fait, à l'intérieur du vaisseau, une position autre que la position la plus favorable avec son axe sensiblement parallèle à l'axe du vaisseau.

La présente invention a pour objet de pallier ces inconvénients en proposant un filtre d'une certaine souplesse pour faciliter la pose, d'une rigidité et d'une stabilité axiale suffisantes pour garantir un bon maintien dans la veine et une qualité de filtration.

Le filtre conforme à l'invention comprend de plusieurs pattes élastiques déployables sensiblement suivant une forme générale conique issues d'une tête, chaque patte étant pourvue vers son extrémité libre d'un patin de centrage et d'appui orienté en position déployée sensiblement parallèlement à la paroi sensiblement cylindrique engendrée par une ligne génératrice parallèle à l'axe de ladite forme conique et décrivant comme ligne génératrice le périmètre d'ouverture de ladite forme conique dans une position normale d'utilisation ; il se caractérise plus particulièrement en ce que lesdites pattes sont constituées, ainsi que lesdits patins, de fils souples et elles sont réunies par groupes de deux pattes adjacentes par une partie de fils repliée en épingle à cheveux qui forme lesdits patins prévus aux extrémités correspondantes desdites pattes.

De cette façon, lorsque le filtre est positionne dans la veine, il présente un appui large qui diminue les traumatismes dus à la pression de contact sur les parois de la veine ; de plus, les patins de centrage et d'appui se plaquent dès le départ contre les parois de la veine en obligeant le filtre à se placer avec son axe sensiblement confondu avec l'axe de la veine.

La structure particulière en fils permet une déformation des pattes et de leurs patins dans plusieurs directions. Cela rend possible le regroupement des pattes sous un faible volume et facilite la pose grâce à des introducteurs fins. En outre, la réunion des pattes deux à deux évite leur emmêlement.

Selon une caractéristique d'un mode de réalisation préféré de l'invention, lesdites parties de fils repliées en épingle à cheveux reviennent sensiblement en arrière en étant dirigées vers la tête du cône.

Selon une autre caractéristique de l'invention, lesdites pattes réunies par groupes de deux pattes adjacentes sont constituées d'un seul fil replié sur lui-même en forme de pince et maintenu ponctuellement au niveau de la tête.

Selon une variante de l'invention, lesdites parties de fils repliées en épingle définissent des aires sensiblement triangulaires. Cette forme particulière ou "triangulation" des fils permet de rapprocher facilement les deux brins de chaque fils. En position repliée, le filtre occupe alors un faible encombrement les deux brins des fils s'étendant sensiblement côte à côte, les uns le long des autres et l'ensemble du faisceau de fils conservant toute sa souplesse et ne risquant pas de s'emméler, lorsque le filtre est lâché dans la veine .

Selon une autre caractéristique de l'invention, les fils souples constituant les pattes sont fixés, à leur extrémité opposée à celle de jonction avec les patins, dans une tête pleine de forme générale ogivale présentant des trous préformés.

L'invention se rapporte en outre, à des moyens d'accrochage du filtre dans la veine, plus précisément des crochets d'ancrage.

On va maintenant donner une description plus détaillée de l'invention, en faisant pour cela référence aux dessins d'accompagnement donnés uniquement à titre d'exemple non limitatif et dans lesquels :
- la figure 1 montre en perspective schématiquement un filtre conformément à l'invention dans son état déployé,
- la figure 2 montre schématiquement le filtre implanté dans un vaisseau,
- la figure 3 est une vue de détail de deux pattes adjacentes réunies entre elles,
- la figure 4 montre la fixation des fils dans une tête pleine, le tiers gauche de la figure étant une vue en coupe,
- la figure 5 montre une vue du dessous de la figure 4, faite sensiblement selon la flèche V de cette figure,
- les figures 6 et 7 montrent deux étapes successives de la formation d'un crochet,
- la figure 8 montre la fixation du crochet sur le fil, la base du crochet étant écrasée autour dudit fil,
- la figure 9 montre en coupe la fixation du crochet, faite sensiblement selon les flèches B,
- la figure 10 montre une variante de fixation du crochet, la base du crochet étant repliée autour du fil,
- la figure 11 montre en coupe suivant les flèches C, la variante de fixation du crochet,
- les figures 12, 13, 14 montrent trois étapes successives de la mise en place du filtre de l'invention dans un vaisseau.

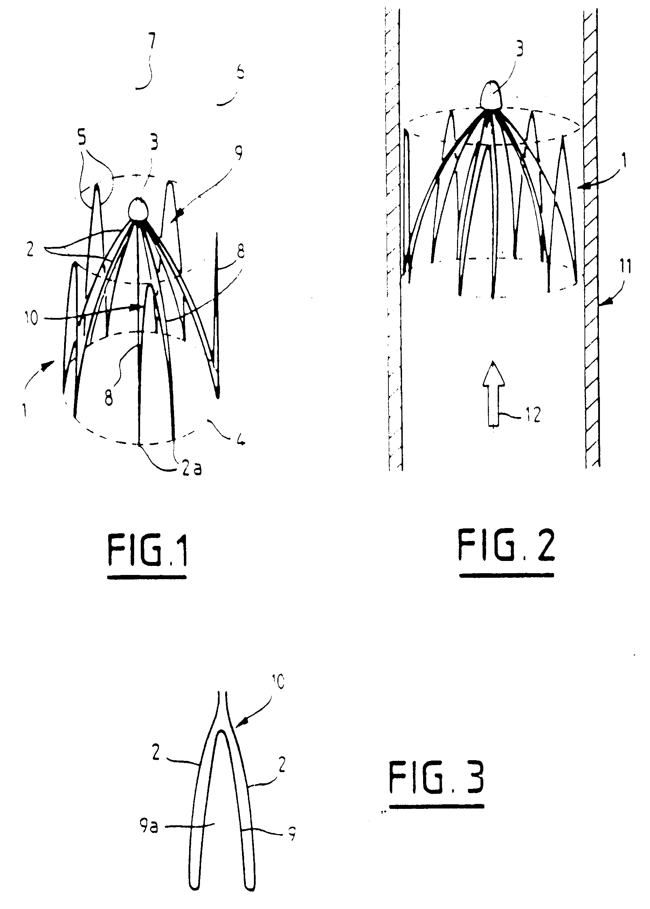

En se référant tout d'abord à la figure 1, on voit donc illustré un filtre 1 en position déployée constitué par des pattes 2, par exemple au nombre de dix, issues d'une tête 3 déployable sensiblement suivant une forme générale conique dont l'ouverture a été marquée en pointillés en 4. Chaque patte est pourvue à son extrémité libre d'un patin de centrage et d'appui 5 revenant sensiblement en arrière de la tête 3 ; c'est-à-dire que ce patin 5 est dirigé à partir de l'extrémité 2a des pattes vers le côté de fermeture du cône que forme le filtre. De manière plus précise, si l'on désigne par 6 le cylindre engendré par une ligne génératrice parallèle à l'axe 7 de la forme conique formée par le filtre 1 en se déplaçant en décrivant la ligne 4, les patins 5 sont dirigés de manière à être sensiblement parallèles à la paroi du cylindre 6.

Conformément à l'invention, les pattes du filtre sont constituées de fils souples 8. Elles pourront notamment être constituées de fils métalliques, par exemple en acier inoxydable de qualité appropriée, telle que par exemple référencée AFNOR K 13C20 N16 Fe15, connu notamment sous le nom de marque déposée "Phynox" ; le diamètre des fils peut être compris entre 2 et 4 dixième de millimètre par exemple 3 ou 3,5 dixième de millimètre.

Ces pattes sont réunies par groupe de deux pattes adjacentes par une partie de fil repliée en épingle à cheveux 9 qui forme lesdits patins. De ce fait, les pattes présentent une forme générale de pince 10, comme illustré ci-après à la figure 3. Avantageusement le filtre comportera au moins six pattes précitées réunies à trois parties de fils repliées en épingle et angulairement réparties afin d'assurer une bonne stabilité axiale du filtre.

La mise en place d'un filtre selon l'invention se fait selon la procédure classique qui est facilitée compte-tenu de la souplesse du filtre.

La figure 2 illustre un filtre 1 implanté en position déployée à l'intérieur d'un vaisseau 11 de manière à pouvoir intercepter les éventuels caillots pouvant y circuler, le sens du flux sanguin étant repéré par la flèche 12. Les patins 5 se plaquent contre la paroi interne de la veine permettant ainsi un autocentrage du filtre dans le vaisseau.

La figure 3 illustre une vue de détail d'une pince 10 constituée par deux pattes 2 adjacentes réunies par une partie de fil repliée en épingle 9. Avantageusement cette partie de fil en épingle délimite une aire 9a sensiblement triangulaire afin d'assurer une plus grande stabilité du filtre implanté.

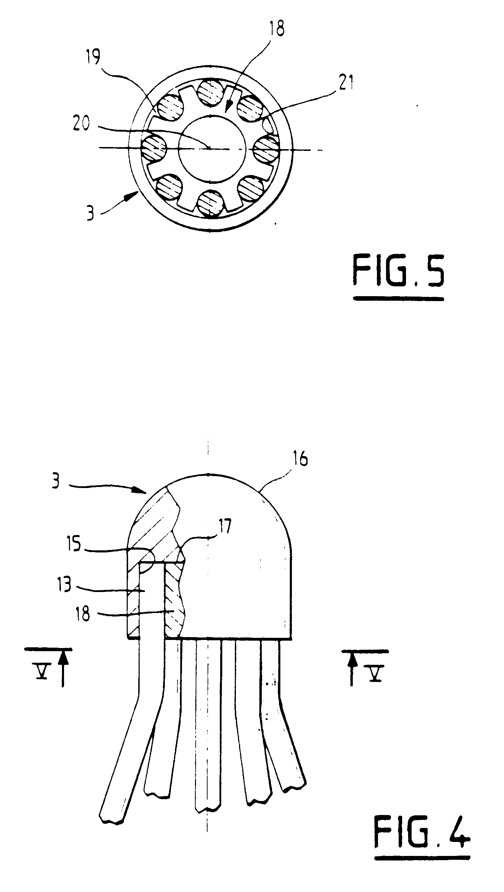

Les figures 4 et 5 illustrent la réunion des extrémités des fils à l'endroit de la tête 3.

D'après la figure 4, les extrémités des fils 13 formant les pattes oposées à celles de jonction avec les patins, sont fixées dans une tête pleine. Les extrémités des fils sont introduites dans autant de trous préformés 15 de la tête. Avantageusement, la tête pleine sensiblement ogivale 16 présente du côté de sa base un alésage cylindrique 17. Les fils sont bloqués dans la tête grâce à une pièce 18 de forme générale cylindrique d'un diamètre et d'une hauteur sensiblement égaux à ceux de l'alésage et présentant des évidements 19 parallèles à l'axe de rotation 20 de ladite pièce et débouchant sur la surface externe de ladite pièce. Ces évidements 19 sont angulairement répartis et les extrémités des fils qui y sont placées sont alors bloquées par la paroi interne 21 de la tête comme illustré à la figure 5.

Les filtres dits définitifs qui sont introduits lorsque le risque de remontée de caillots est constant et qui reste donc en place pendant la durée de la vie du patient sont munis de moyens d'accrochage pour prévenir toute migration du filtre dans le vaisseau.

Les filtres selon l'invention sont pourvus de tels moyens d'accrochage devant pénétrer légèrement dans la paroi du vaisseau. En effet, certains au moins de leurs patins sont pourvus de crochets d'ancrage.

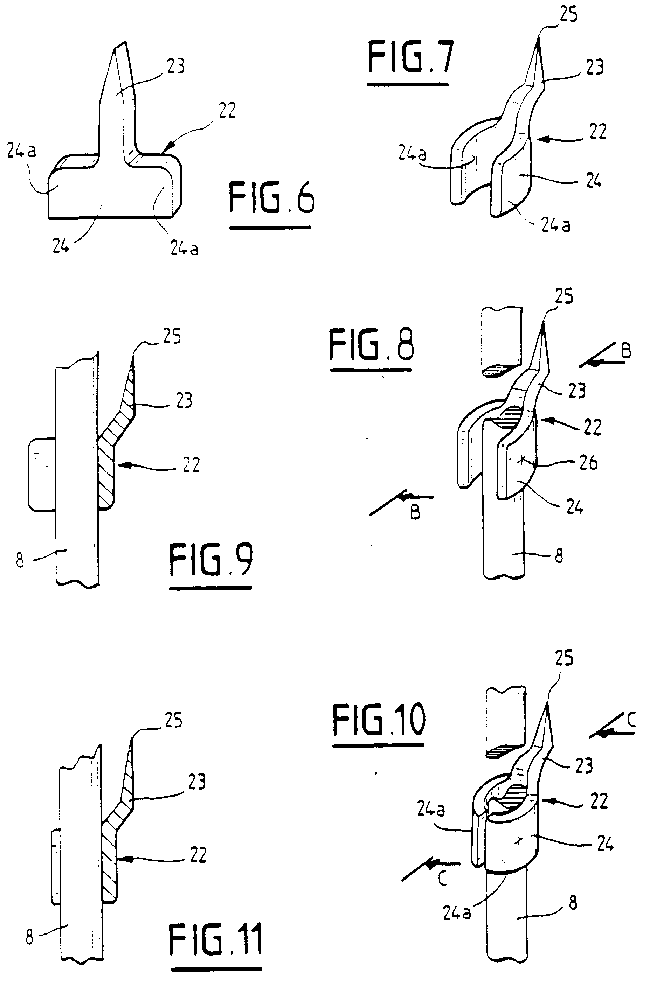

En se référant aux figures 6 et 7, on décrira la constitution et le procédé de fabrication de ces crochets d'ancrage.

On part d'une plaque mince par exemple métallique dans laquelle on découpe l'ébauche d'un crochet 22 comme montré à la figure 6 sensiblement sous la forme d'un T dont la hampe est une partie effilée 23.

A ladite ébauche ainsi constituée, on fait subir une opération de formage comme illustré à la figure 7. Tout d'abord on déforme la partie effilée sensiblement en V en l'écartant du plan de l'ébauche. On déforme également la barre 24 du T en repliant ses deux bras 24a jusqu'à ce qu'ils soient sensiblement parallèles. Après quoi on affute ladite partie effilée 23 de façon que son extrémité forme une pointe 25.

On se reportera maintenant aux figures 8 à 11 pour décrire la mise en place du crochet sur un bras du fil.

Les figures 8 et 9 illustrent une première technique de fixation des crochets sur les fils. Le crochets 22 placé sur le fil 8 est fixé en écrasant les bras 24a de la barre 24 autour du fil en des points 26, comme cela est montré à la figure 8.

Comme illustré à la figure 9 qui montre une vue en coupe de la figure 8, les bras 24a restent libres et parallèles.

Les figures 10 et 11 illustrent une seconde technique possible de fixation des crochets. D'après la figure 10, le crochet 22 est placé sur le fil 8 les deux bras 24a étant ensuite repliés autour dudit fil.

A la figure 11 on a illustré une vue en coupe de la figure 10 montrant le repliage des bras 24a dont les extrémités sont rapprochées.

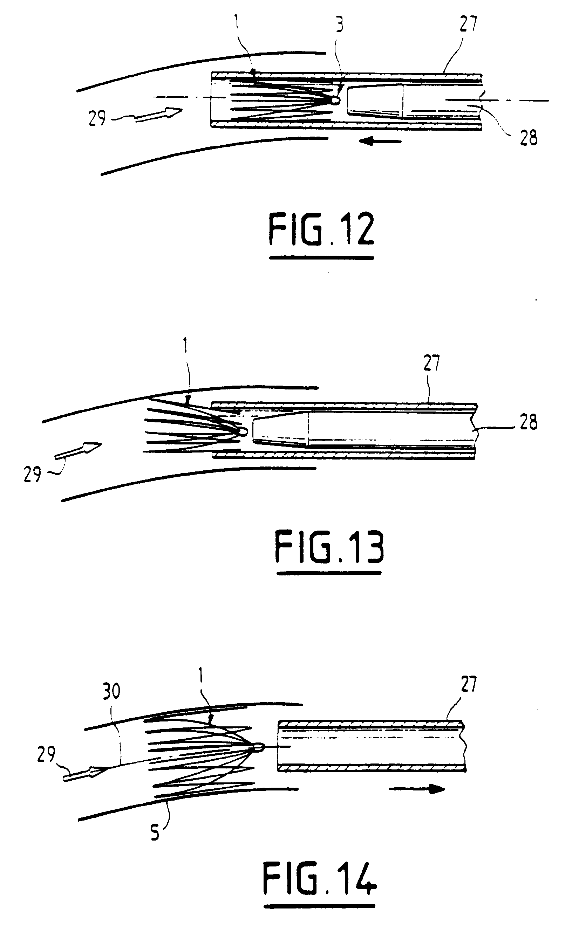

Sur les figures 12, 13, 14 on a illustré schématiquement un mode d'introduction du filtre selon l'invention dans un vaisseau.

La mise en place du filtre est faite à travers un tube d'introduction 27 et désigné habituellement dans la technique "Desilet".

A la figure 12, le filtre 1 est poussé à l'intérieur du tube 27 grâce à un poussoir 28. On remarquera que le filtre, en position repliée, est placé de telle sorte que sa tête 3 sorte en dernier, le tube 27 ayant été introduit à contre sens du flux sanguin indiqué par la flèche repérée 29.

A la figure 13, le filtre 1 apparaît sortant du tube et présentant déjà une forme sensiblement conique.

A la figure 14, on aperçoit le filtre 1 un instant plus tard. Les pattes sont entièrement déployées et leurs patins 5 définissent une enveloppe sensiblement cylindrique quasiment coaxiale à l'axe 30 du vaisseau en appuyant sur ses parois assurant l'autocentrage du filtre dans la veine.

Le tube 27 peut alors être retiré par sa voie d'accès.

La mise en place décrite ci-dessus correspond à la pose d'un filtre par la voie jugulaire. Si la pose se fait par la voie fémorale, le filtre est lâché dans la veine en sens opposé, tête en avant, mais là encore les pattes triangulées qui le constituent assurent son centrage.

Il va de soi que la présente invention n'a été décrite qu'à titre purement explicatif et nullement limitatif et que toute modification utile pourra y être apportée sans sortir de son cadre tel que défini par les revendications ci-après. Ainsi selon une variante, les filtres décrits peuvent parfaitement être utilisés comme filtres temporaires, et dans ce cas ils sont dépourvus des crochets d'ancrage.

Selon une autre variante, lesdites pinces peuvent toutes être formées à partir d'un seul fil souple convenablement conformé et maintenu sensiblement ponctuellement au niveau de la tête.

## Revendications

1. Filtre destiné à être placé sur le trajet sanguin en particulier pour la retenue de caillots de sang comprenant plusieurs pattes (2) élastiques déployables sensiblement suivant une forme générale conique issue d'une tête (3), chaque patte étant pourvue vers son extrémité libre d'un patin de centrage et d'appui (5) orienté en position déployée sensiblement parallèlement à la paroi sensiblement cylindrique (6) engendrée par une ligne génératrice parallèle à l'axe (7) de ladite forme conique et décrivant comme ligne génératrice le périmètre d'ouverture (4) de ladite forme conique dans une position normale d'utilisation caractérisée en ce que lesdites pattes sont constituées, ainsi que lesdits patins, de fils souples et elles sont réunies par groupes de deux pattes adjacentes par une partie de fil repliée en épingle à cheveux qui forme lesdits patins prévus aux extrémités correspondantes desdites pattes.

2. Filtre selon la revendication 1 caractérisé en ce que lesdites parties de fil repliées en épingle à cheveux reviennent sensiblement en arrière en étant dirigées vers la tête du cône.

3. Filtre selon la revendication 1 ou la revendication 2 caractérisé en ce que lesdites pattes réunies par groupe de deux pattes adjacentes sont constituées d'un seul fil replié sur lui-même en forme de pince et maintenu ponctuellement au niveau de la tête.

4. Filtre selon la revendication 3 caractérisé en ce que lesdites pinces sont toutes formées à partir d'un seul fil souple convenablement conformé et maintenu sensiblement ponctuellement au niveau de ladite tête.

5. Filtre selon l'une quelconque des revendications précédentes caractérisé en ce que lesdites parties de fils repliées en épingle (9) définissent des aires sensiblement triangulaires (9a).

6. Filtre selon l'une quelconque des revendications précédentes caractérisé en ce qu'il comporte au moins six pattes précitées réunies à trois parties de fils repliées en épingle et réparties angulairement autour de l'axe (7) précité du filtre.

7. Filtre selon l'une quelconque des revendications précédentes caractérisé en ce que lesdits fils souples constituant lesdites pattes sont fixés, à leur extrémité opposée à celle de jonction avec lesdits patins, dans une tête pleine de forme générale ogivale présentant des trous préformés.

8. Filtre selon l'une quelconque des revendications 1 à 7 caractérisé en ce que sur certain au moins des patins précités sont prévus des crochets d'ancrage.

9. Filtre selon la revendication 8 caractérisé en ce que les crochets sont pourvus de parties métalliques repliées et fixées sur lesdits patins.

## Claims

1. Filter to be placed in the path of blood, particularly for retaining blood clots, comprising a plurality of resilient legs (2) which can be spread out substantially in a generally conical shape extending from a head (3), each leg being provided, towards its free end, with a locating and bearing foot (5) which, in the spread-out position, is oriented substantially parallel to the substantially cylindrical wall (6) generated by a generatrix parallel to the axis (7) of the said conical shape and describing, as a generatrix, the perimeter of the opening (4) of the said conical shape, in a normal position of use, characterized in that the said legs, as well as the said feet, are made of flexible wire and the legs are joined together in groups of two adjacent legs by a portion of wire which is bent like a hairpin and forms the said feet provided at the corresponding ends of the said legs.

2. Filter according to Claim 1, characterized in that the said portions of wire which are bent like hairpins turn substantially backwards, being directed towards the head of the cone.

3. Filter according to Claim 1 or Claim 2, characterized in that the said legs which are joined together in groups of two adjacent legs are made of a single wire bent onto itself in the shape of pincers and held at a point at the level of the head.

4. Filter according to Claim 3, characterized in that all of the said pincers are formed from a single flexible wire suitably shaped and held substantially at a point at the level of the said head.

5. Filter according to any one of the preceding claims, characterized in that the said portions of wire which are bent like hair-pins (9) define substantially triangular areas (9a).

6. Filter according to any one of the preceding claims, characterized in that it includes at least six of the above-mentioned legs joined to three portions of wire bent like hairpins and distributed angularly around the aforesaid axis (7) of the filter.

7. Filter according to any one of the preceding claims, characterized in that the said flexible wires forming the said legs are fixed, at their opposite ends to those joined to the said feet, in a solid head of generally ogival shape having preformed holes.

8. Filter according to any one of Claims 1 to 7, characterized in that anchorage hooks are provided on at least some of the aforesaid feet.

9. Filter according to Claim 8, characterized in that the hooks are provided with metal portions which are bent and fixed on the said feet.

## Patentansprüche

1. Filter, der dazu bestimmt ist, an der Blutbahn, insbesondere für das Zurückhalten von Blutkoagulaten, angebracht zu werden, mit mehreren elastischen, entfaltbaren Stützen (2), die im wesentlichen eine von einem Kopf (3) ausgehende allgemeine konische Form haben, wobei jede Stütze an ihrem freien Ende mit einer Zentrier- und Stützkufe (5) versehen ist, die in entfalteter Position im wesentlichen parallel zu einer im wesentlichen zylindrischen Wand (6) gerichtet ist, welche durch eine zu der Achse (7) der konischen Form parallele Erzeugende erzeugt ist, und als Erzeugende den Öffnungsumfang (4) der konischen Form in einer normalen Benutzungsposition beschreibt, **dadurch gekennzeichnet**, daß die Stützen, sowie die Kufen, aus weichen Fäden gebildet und in Gruppen von zwei angrenzenden Stützen durch einen Fadenteil verbunden sind, der als Haarnadel zurückgebogen ist, welcher die Kufen bildet, die an den entsprechenden Enden der Stützen vorgesehen sind.

2. Filter nach Anspruch 1, dadurch gekennzeichnet, daß die als Haarnadel zurückgebogenen Fadenteile im wesentlichen wieder nach hinten kommen, wobei sie zum Kopf des Kegels gerichtet sind.

3. Filter nach Anspruch 1 oder Anspruch 2, dadurch gekennzeichnet, daß die in Gruppen von zwei angrenzenden Stützen vereinigten Stützen aus einem einzigen Faden gebildet sind, der auf sich selbst in der Form einer Zange zurückgebogen und punktuell an dem Kopf gehalten ist.

4. Filter nach Anspruch 3, dadurch gekennzeichnet, daß die Zangen alle, von einem einzigen weichen Faden ausgehend, gebildet sind, der geeignet geformt und im wesentlichen punktuell an dem Kopf gehalten ist.

5. Filter nach einem der vorhergehenden Ansprüche, dadurch gekennzeichnet, daß die als Nadel (9) gebogenen Fadenteile im wesentlichen dreieckige Flächen (9a) bestimmen.

6. Filter nach einem der vorhergehenden Ansprüche, dadurch gekennzeichnet, daß er mindestens sechs zuvor genannte Stützen aufweist, die mit drei als Nadel zurückgebogenen Fadenteilen verbunden und winkelig um die Achse (7) des vorhergenannten Filters verteilt sind.

7. Filter nach einem der vorhergehenden Ansprüche, dadurch gekennzeichnet, daß die weichen Faden, welche die Stützen bilden, an ihrem Ende, das dem der Verbindung mit den Kufen gegenüberliegt, an einem massiven, allgemein spitzbogenförmigen Kopf befestigt sind, der vorgeformte Löcher aufweist.

8. Filter nach einem der Ansprüche 1 bis 7, dadurch gekennzeichnet, daß mindestens auf einigen der vorgenannten Kufen Verankerungshaken vorgesehen sind.

9. Filter nach Anspruch 8, dadurch gekennzeichnet, daß die Haken mit metallischen, zurückgebogenen Teilen versehen und an den Kufen befestigt sind.
